# EUROPÄISCHE PATENTSCHRIFT

(11) **EP 0 776 197 B1**
(45) Veröffentlichungstag und Bekanntmachung des Hinweises auf die Patenterteilung: **11.11.1998**
(21) Anmeldenummer: 95929104.8
(22) Anmeldetag: 12.08.1995
(51) Int. Cl.: A61K 9/70

(54) **ESTRADIOL-TTS MIT WASSERBINDENDEN ZUSÄTZEN**
OESTRADIOL-CONTAINING TRANSDERMAL THERAPEUTIC SYSTEM COMPRISING HYDROPHYLIC ADDITIVES
SYSTEME THERAPEUTIQUE TRANSDERMIQUE CONTENANT DE L' STRADIOL ET DES ADDITIFS HYDROPHILES

(30) Priorität: 20.08.1994 DE 4429664
(43) Veröffentlichungstag der Anmeldung: 04.06.1997
(73) Patentinhaber: LTS LOHMANN Therapie-Systeme GmbH, 56567 Neuwied (DE)
(72) Erfinder: HORSTMANN, Michael, D-56564 Neuwied (DE); MÜLLER, Walter, D-56564 Neuwied (DE); FRANKE, Hanshermann, D-56566 Neuwied (DE); PONTZEN, Thomas, D-56575 Wei enthurm (DE)
(74) Vertreter: Flaccus, Rolf-Dieter, Dr.
(86) Internationale Anmeldenummer: EP9503202
(87) Internationale Veröffentlichungsnummer: WO9605815

(56) Entgegenhaltungen:
- DE-C- 4 223 360
- DE-C- 4 237 453

## Beschreibung

Die Erfindung betrifft ein transdermales therapeutisches System mit dem Wirkstoff Estradiol und gegebenenfalls weiteren Wirkstoffen sowie mit einem wasserbindenden Zusatzstoff, mit geschichtetem Aufbau einer wirkstoff- und feuchtigkeitsundurchlässigen Rückschicht, einer wirkstoffhaltigen Matrixschicht und fallweise einer ablösbaren die Matrixschicht abdeckenden Schutzschicht.

Transdermale Therapeutische Systeme (TTS) sind in der arzneilichen Therapie einer Reihe von Erkrankungen bereits im Markt eingeführt.

Unter dem Begriff "Estradiol" soll im folgenden die wasserfreie Substanz des 17-β-Estradiols in gelöster oder kristalliner (Anhydrat) Form verstanden werden.

TTS mit dem Wirkstoff Estradiol sind inzwischen als Therapeutikum für Wechseljahrsbeschwerden, neuerdings auch gegen Osteoporose im Handel und bewähren sich erfolgreich in der Therapie.

Ein Nachteil der dem Stand der Technik entsprechenden Systeme ist die nicht ausreichende Permeationsfähigkeit des Wirkstoffs durch die Haut, die auch durch zahlreiche galenische Maßnahmen des TTS-Aufbaues (Einsatz mehrschichtiger Systeme, Verwendung von Steuermembranen, Variation der Wirkstoffkonzentration, Modifikation der Grundpolymeren etc.) nicht über eine gewisse Grenze, den sogenannten "Sättigungsfluß", hinaus steigerbar ist. Diese Feststellung, daß der transdermale Fluß eines Wirkstoffes aus der festen, fein verteilten Phase heraus auch bei Einsatz stärker lösender Vehikel prinzipiell nicht weiter steigerbar ist, findet sich bereits in den wegweisenden Arbeiten von Higuchi, z.B. T. Higuchi: Physical Chemical Analysis of percutaneous process from creams and ointments. J. Soc. Cosmetic Chem. 11, S. 85-97 (1960).

In der EP 0 421 454 beschriebene Systeme enthalten Estradiol in einem Acyrlatpolymer unter Zusatz von "Kristallisationshemmern" und klebrigmachenden Harzen. Quellstoffe sind zum Schutz vor vorzeitigem Klebkraftverlust enthalten. Zudem gibt es für viele Wirkstoffe die Möglichkeit, dem TTS bei der Herstellung sogenannte "Enhancer" zuzusetzen. Es handelt sich hier um in der Regel flüssige Zusatzstoffe, die die Resorptionseigenschaften der menschlichen Haut verbessern und damit die Aufnahme des Wirkstoffes aus einer genügend kleinen TTS-Fläche heraus ermöglichen. Speziell leicht flüchtige Enhancer wie das zum Beispiel für den Wirkstoff Estradiol verwendete Ethanol bergen Probleme einer starken Erweichung der Klebschichten des TTS und machen vor allem weitere, raumfordernde Kompartimente im System notwendig, die das TTS unakzeptabel dick werden lassen. Schließlich birgt jeder weitere nichtpolymere Zusatzstoff auch die Gefahr von unverträglichkeiten auf der Haut, unter Umständen auch von Sensibilisierungen. Unter Zusatz bestimmter weniger flüchtiger, meist aber auch weniger aktiver Enhancer (z.B. Glycerinester, cyclische Amide, Eucalyptol) ist zwar die Herstellung von Matrixsystemen möglich, die den Wirkstoff und die resorptionsverstärkende Komponente in einer oder mehreren monolithischen Schichten enthalten.
US 4 863 738 stellt ein Beispiel von vielen dar, in denen die Applikation von Wirkstoffen, z.B. Estradiol, zusammen mit einem bestimmten Enhancer (hier Glycerinmonooleat) in einer beliebigen TTS-Matrix in beliebiger Konzentration beansprucht wird.
Nach dem Stand der Technik ist auch mit solchen TTS keine befriedigende Therapie möglich, da entweder die wirksamen Enhancer zu schlecht hautverträglich sind oder die Systeme wegen des noch immer zu geringen Flusses durch die Haut unakzeptabel große Flächen benötigen.

Eine andere Möglichkeit, den Wirkstofffluß durch die Haut zu steigern, besteht darin, mehr Wirkstoff im TTS molekulardispers aufzulösen als der Sättigungslöslichkeit entspricht. Mit Übersättigung solcher Systeme steigt im gleichen Maße die Permeationsgeschwindigkeit der Haut an. Da solche Zustände jedoch thermodynamisch instabil sind, sind solche Arzneiformen nicht lagerstabil; es findet eine zeitlich nicht vorhersehbare Rekristallisation von Wirkstoffteilchen statt, sodaß die Flußrate durch die Haut nach und nach auf Sättigungsflußniveau abfällt und damit je nach Ausgangskonzentration ein großer Teil der initial vorhandenen therapeutischen Aktivität verloren geht.

Dieser Vorgang ist eine Besonderheit des Estradiols.

Estradiol liegt bei Raumtemperatur und gewöhnlicher relativer Luftfeuchtigkeit (20-60% relativer Feuchte) nicht in einer der beiden bekannten kristallwasserfreien Modifikationen (I und II) vor, sondern als Semihydrat (Busetti u. Hospital, Acta Cryst. 1972, B28, 560). Wegen der schichtförmigen, über Wasserstoffbrücken stabilisierten Struktur und der Diffusionsdichtigkeit des Kristallverbundes läßt sich das Semihydrat kurzzeitig unzersetzt bis zu Temperaturen von ca. 170°C erhitzen (Kuhnert-Brandstätter u. Winkler (1976) Scientia Pharmaceutica 44 (3), 177-190). Durch Mikronisieren kann jedoch auf dem Wege der Kristalloberflächenvergrößerung bereits bei ca. 120°C quantitiv die wasserfreie Form erhalten werden. Nach eigenen Beobachtungen findet bei sehr langsamem Erwärmen (o,2-1 K/min) und besonders feinkristalliner Substanz die Umwandlung sogar bereits bei etwa 90°C statt.

Andererseits weist Estradiol mit geringer werdendem Wasserdampfpartialdruck höhere Löslichkeiten in einigen Polymeren, speziell auch Polyacrylaten auf. Da mit höherer Konzentration unter sonst gleichen Bedingungen der Diffusionsfluß durch die Haut nach dem Fickschen Gesetz zunimmt, ist eine solche Konzentrationssteigerung bei transdermalen therapeutischen Systemen sehr erwünscht. Jedoch reicht bereits das mit dem Estradiol-semihydrat eingebrachte Wasser aus, um nach und nach ein erneutes Auskristallisieren als Estradiol-semihydrat aus der Lösung auszulösen (Kuhnert-Brandstätter u. Winkler (1976) Scientia Pharmaceutica 44 (3), 177-190). Nach dem Auskristallisieren fällt mit der geringer werdenden Konzentration auch die Flußrate aus dem System an die Haut stark ab.

Diese Umstände berücksichtigend sind transdermale Systeme bekannt, welche durch genaue Konzentrationseinstellung knapp unter der Sättigungslöslichkeit des Estradiol-Anhydrates (DE-PS 42 37 453) oder durch Verwendung teilweise ungelösten, dispergierten Estradiol-Anhydrates (DE-PS 42 23 360) eine pharmakotherapeutisch befriedigende Lösung anbieten.

Auch bei Berücksichtigung dieses Standes der Technik ist es wichtig, während Herstellung und Lagerung eines Estradiol-TTS eine ausreichend niedrige Luftfeuchtigkeit aufrechtzuerhalten, damit eine großflächige Ausfällung des schwerlöslichen Estradiol-semihydrates vermieden wird. Zu diesem Zweck kann zwar grundsätzlich eine wasserdichte Verpackung mit geringer Wasserdampfdurchlässigkeit verwendet werden. Bei der geringen Konzentration an Estradiol, die in heutigen TTS enthalten ist, reichen jedoch sehr geringe Feuchtigkeitsmengen zur Ausfällung des Estradiol-semihydrates aus. Sind beipsielsweise 2 mg waEstradiol (wasserfrei) in einem TTS gelöst vorhanden, ist bereits eine Menge von 66,1 µg Wasser zur vollständigen Ausfällung in der Lage. Es ist dementsprechend mit konventionellen Packmitteln sehr schwierig, über Lagerzeiten von mehreren Jahren den Zutritt solch geringer Feuchtigkeitsmengen auszuschließen.

DE 42 37 453 schlägt bereits die Verwendung von Trockenmitteln in der lagerungsfertigen Packung vor, es finden sich jedoch keine Angaben zur Verwendung des wasserbindenden Zusatzstoffes als Bestandteil der Matrix.

Aufgabe dieser Erfindung ist daher, ein transdermales therapeutisches System mit Estradiol anzugeben, das in der Verpackung während der Lagerdauer einen Schutz des Systems vor Ausfällung des Estradiol-Semihydrates bereitstellt und bedarfsweise für eine Matrix geeignet ist, in welcher mehr Wirkstoff molekulardispers aufgelöst enthalten ist als der Sättigungslöslichkeit entspricht.

Diese Aufgabe wird bei einem transdermalen therapeutischen System der im Oberbegriff von Anspruch 1 genannten Art mit der Erfindung dadurch gelöst, daß der wasserbindende Zusatzstoff ein Bestandteil der Matrix ist.

Die erfindungsgemäße Einbindung wasserbindender mineralischen Bestandteile in der Substanz der Matrix kann auf unterschiedliche Weise in einem TTS realisiert werden: Die einfachste Form ist ein einschichtiges Matrixsystem, dessen Matrix von ihrer Funktion her gleichzeitig haftklebend ist und damit eine zusätzliche Klebschicht überflüssig macht.

Durch die in der Matrix enthaltenen gelösten oder in der Regel in fester Phase dispergierten mineralischen Bestandteile wird über die Lagerdauer eine derart niedrige Gleichgewichtsfeuchte gewährleistet, daß eine Fällung von Estradiol-Semihydrat unmöglich wird.
Soll die direkte Berührung von der Haut mit mineralischen Partikeln vermieden werden, wird nur die Matrix mit wasserbindenden mineralischen Bestandteilen ausgestattet und durch Kaschieren eine hautzugewandte Klebschicht, die frei von partikulärem Estradiol ist, aufgebracht.

Wird zwischen einer solchen Matrix, enthaltend Estradiol und wasserbindende mineralische Bestandteile, und der Klebschicht eine für Estradiol schwer durchlässige Membran eingebracht, erreicht man eine modifizierte Wirkstoffabgabe.

Als Grundmaterial können neben den weitverbreiteten und für Estradiol gut einsetzbaren Acrylsäureestercopolymeren auch andere Polymere, wie Polyisobutylen, Polyvinylacetat und Copolymere, synthesekautschuk, Silicone verwendet werden.

Kennzeichnend für erfindungsgemäße transdermale therapeutische System ist in jedem Falle die Anwesenheit von wasserbindenden mineralischen Bestandteilen in der Matrix. Dabei ist der genaue Anteil dieser Zusatzstoffe in der Matrix ausreichend hoch zu wählen, da hiermit die Gesamt-Feuchtigkeitsbindung im System zunimmt. Die genaue Menge an Zuschlagsstoffen ergibt sich in für den Fachmann nachvollziehbarer Weise aus der Bindungsfähigekit des Bestandteils für Wasser. Da die Bindungskapazität der allermeisten der nachstehend beispielhaft erwähnten Stoffe für Wasser generell über den von Estradiol liegt, kann als Mindestmenge ein Zusatz in der Größenordnung des enthaltenen Estradiols gelten - etwa 1 bis 2 Prozent. Die Obergrenze wird durch mechanische Größen wie Fließfähigkeit der Matrix, Klebkraft und Verarbeitbarkeit bestimmt; in der Regel wird ein Anteil von 10 bis 50 Gewichtsprozenten, bevorzugt zwischen 20 und 35 Gewichtsprozent, angestrebt.

Als wasserbindende mineralische Bestandteile können z.B. Zinkoxid, Siliciumdioxid, Silicagel (auch in modifizierter, z.B. hydrophobierter Form), Talkum, Phophorpentoxid, Aluminiumoxid, Aluminiumphophat, Magnesiumoxid und -hydroxid, Calciumoxid und -hydroxid, Karolin, Molekularsiebe, Natriumoxid, Calcium- und Magnesiumcarbonat, Magnesiumsulfat, Calciumsulfat, Kupfersulfat, Manganoxide, Silikate, Aluminate oder Magnesiumperchlorat verwendet werden.

Dabei ist zu beachten, daß hier sowohl chemisch-reaktive Prozesse, zum Beispiel die Reaktion von Magnesiumoxid zu Magnesiumhydroxid wie auch die physikalische Übergänge, wie zum Beispiels der Einschluß von Kristallwasser in zunächst wasserfreies Natriumsulfat, Calciumsulfat oder Calciumchlorid, oder auch reine, nicht stöchiometrische Hygroskopie oder Adsorptivität ausgenutzt werden können, die dem Fachmann als solche allgemein bekannt sind.

Bevorzugt zum Einsatz kommen Stoffe, die bei niedriger Luftfeuchtigkeit (im Bereich unter etwa 5% realtiver Luftfeuchtigkeit) eine hohe Bindungskapazität für Wasser aufweisen. Weiterhin ist ein hohes Maß an physiologischer Verträglichkeit erwünscht.
Diese Bedingungen sind erfreulicherweise bei zahlreichen Hydraten von Salzen der Erdalkali- und Alkalimetalle erfüllt, wie zum Beispiel bei Calcium- und Mangnesiumcarbonat, Calcium-, Natrium- und Magnesiumsulfat oder auch vielen Silikaten, Aluminaten, Boraten und Phosphaten der Alkali-(bevorzugt Natrium, Kalium und Lithium) und Erdalkalimetalle (Calcium und Magnesium).

Weitere Ausgestaltungen der Erfindung sind entsprechend den Unteransprüchen vorgesehen.

### Beispiele:

### Beispiel 1: Herstellung eines erfindungsgemäßen Systems

2,0 g 17-β-Estradiol-semihydrat, mikronisiert
60,0 g Cariflex^{R} TR 1107 (Styrol-Isopren-Styrol-Blockcopolymer)
120,0 g Staybelite Ester 5E (thermoplastisches Esterharz aus Kolophoniumderivaten)
50 g dickflüssiges Paraffin
50 g Calciumsulfat-Dihydrat
werden in einem evakuierbaren Kneter bei 130°C aufgeschmolzen und durch Kneten innerhalb von zehn Stunden in eine äußerlich homogene Form gebracht. Unter Vakuum (weniger als 0,02 bar) wird nun eine Stunde lang auf 165°C erhitzt und dabei unter Kneten Kristallwasser aus Wirkstoff und Calciumsulfat ausgetrieben.

Die schmelze wird auf 120°C abgekühlt und anschließend auf einer kontinuierlich arbeitenden Beschichtungsanlage auf 100 Mikrometer dicke, siliconisierte Polyesterfolie so beschichtet, daß ein Schichtflächengewicht von 200 g/m² resultiert.
Anschließend wird 15 Mikrometer dicke Polyesterfolie luftblasenfrei unter Walzendruck auf die noch warme Schicht aufgelegt (zukaschiert).
Durch Stanzung mit Henkellocheisen werden transdermale Systeme von 16 cm² erhalten.

### Beispiel 2: Herstellung eines erfindungsgemäßen Systems

3,0 g 17-β-Estradiol-semihydrat, mikronisiert
400,0 g Acrylsäureestercopolymerlösung (Festkörpergehalt 50% g/g)
70,0 g Calciumsulfat, wasserfrei ("Anhydrit")
werden bei Raumtemperatur in einem zylindrischen Glasgefäß bis zum Erreichen einer gleichmäßigen Suspension gerührt und anschließend mit einer Spaltbreite von 500 Mikrometern auf 100 MIkrometer dicke, siliconisierte Polyesterfolie beschichtet. Der Ausstrich wird je 10 Minuten lang bei 25°C, bei 50°C, bei 80°C und bei 95°C getrocknet. Sofort wird 15 Mikrometer dicke Polyesterfolie luftblasenfrei unter Walzendruck auf die getrocknete Schicht aufgelegt (zukaschiert).
Durch Stanzung mit Henkellocheisen werden transdermale Systeme von 10 cm² erhalten, die in Verbundpackstoff aus Papier/Aluminiumfolie/Heißsiegelschicht unter Zugabe einer Trockentablette, enthaltend 0,3 g Calciumsulfat (welches zuvor bei 180°C vorgetrocknet wurde) verpackt werden.

## Patentansprüche

1. Transdermales therapeutisches System mit dem Wirkstoff Estradiol und gegebenenfalls weiteren Wirkstoffen und gegebenenfalls wasserbindenden Zusatzstoffen, mit geschichtetem Aufbau einer wirkstoff- und feuchtigkeitsundurchlässigen Rückschicht, einer wirkstoffhaltigen Matrixschicht und fallweise einer ablösbaren Schutzschicht,
**dadurch gekennzeichnet,**
daß die Matrix den Wirkstoff als wasserfreies Estradiol zusammen mit einam wasserbindenden Zusatzstoff enthält, durch den eine die Ausfällung von Estradiol-semihydrat unterbindende Gleichgewichtsfeuchte gewährleistet wird.

2. Transdermales therapeutisches System nach Anspruch 1, dadurch gekennzeichnet, daß die Matrix mehrere wirkstoffhaltige Schichten aufweist und wenigstens eine dieser Schichten einen pharmazeutisch verträglichen, wasserbindenden mineralischen Zusatzstoff enthält.

3. Transdermales therapeutisches System nach Anspruch 1 oder 2, dadurch gekennzeichnet, daß das Matrixmaterial den wasserbindenden Zusatzstoff in fein dispergierter Suspension enthält.

4. Transdermales therapeutisches System nach einem oder mehreren der Ansprüche 1 bis 3, dadurch gekennzeichnet, daß der wasserbindende Zusatzstoff ein Mineral ist.

5. Transdermales therapeutisches System nach einem oder mehreren der Ansprüche 1 bis 4, dadurch gekennzeichnet, daß der wasserbindende Zusatzstoff das Anhydrat eines Erdalkali- oder Alkalimetallsalzes ist.

6. Transdermales therapeutisches System nach einem oder mehreren der Ansprüche 1 bis 4, dadurch gekennzeichnet, daß der wasserbindende Zusatzstoff das Semihydrat oder Anhydrat ("Anhydrit") des Calciumsulfates ist.

7. Transdermales therapeutisches System nach einem oder mehreren der Ansprüche 1 bis 6, dadurch gekennzeichnet, daß das Estradiol als Dispersion eines wasserfreien Kristallisates vorliegt.

8. Transdermales therapeutisches System nach einem oder mehreren der Ansprüche 1 bis 7, dadurch gekennzeichnet, daß im Matrix-Grundmaterial mehr Estradiol molekulardispers aufgelöst enthalten ist als der Sättigungslöslichkeit entspricht.

9. Transdermales therapeutisches System nach einem oder mehreren der Ansprüche 1 bis 8, dadurch gekennzeichnet, daß der Anteil des mineralischen wasserbindenden Zusatzstoffes am gesamten Matrixmaterial zwischen 1 und 40 Gew.-% und bevorzugt 20 Gew.-% beträgt.

10. Transdermales therapeutisches System nach einem oder mehreren der Ansprüche 1 bis 9, dadurch gekennzeichnet, daß das Matrix-Grundmaterial ein Polymer ist, das unter wasserfreien Bedingungen eine Löslichkeit für den Wirkstoff Estradiol zwischen 0,4 und 3,0 % (g/g) aufweist.

11. Transdermales therapeutisches System nach Anspruch 10, dadurch gekennzeichnet, daß das Matrix-Grundmaterial ein Polyacrylat-Polymer ist.

12. Verfahren zur Herstellung des transdermalen therapeutischen Systems nach einem oder mehreren der vorangehenden Ansprüche, gekennzeichnet durch die Arbeitsschritte :
- in einer Lösung, Dispersion oder Schmelze des Matrix-Grundmaterial wird eine Suspension von Estradiol-Semihydrat und wasserbindendem mineralischem Zusatzstoff bereitet
- mit der Suspension wird auf einem folienförmigen Trägermaterial eine Schicht aufgetragen
- in der Schicht wird durch Erhitzen auf 90 bis 175°C eine Umwandlung von Estradiol-Semihydrat in wasserfreies Estradiol durchgeführt.

13. Verfahren nach Anspruch 12, dadurch gekennzeichnet, daß die getrocknete Schicht auf 90 bis 200°C solange erhitzt wird, bis eine Umwandlung der wasserhaltigen Form des mineralischen wasserbindenden Zusatzstoffes in seine wasserfreie Form erfolgt ist.

14. Verfahren nach einem der Ansprüche 12 oder 13, dadurch gekennzeichnet, daß das transdermale therapeutische System in einem wasserdampfdichten Packmittel verpackt wird, wobei dieses fallweise ein zusätzliches Trocknungsmittel enthält.

## Claims

1. A transdermal therapeutic system with the active substance estradiol and, optionally, further active substances and, optionally, water-binding additives, having a layered structure comprising a backing layer which is impermeable to active substances and moisture, an active substance-containing matrix layer, and, optionally, a removable protective layer,
**characterised in that**
the matrix contains the active substance as water-free estradiol, together with a water-binding additive, said water-binding additive ensuring an equilibrium moisture which prevents the precipitation of estradiol semihydrate.

2. The transdermal therapeutic system according to claim 1 characterized in that the matrix exhibits several active substance-containing layers and that at least one of these layers comprises a pharmaceutically acceptable, water-binding mineral additive.

3. The transdermal therapeutic system according to claim 1 or 2 characterized in that the matrix material comprises the water-binding additive substance in finely dispersed suspension.

4. The transdermal therapeutic system according to one or several of claims 1 to 3 characterized in that the water-binding additive substance is a mineral.

5. The transdermal therapeutic system according to one or several of claims 1 to 4 characterized in that the water-binding additive is the anhydrate of an alkaline-earth or alkali metal salt.

6. The transdermal therapeutic system according to one or several of claims 1 to 4 characterized in that the water-binding additive is the semihydrate or anhydrate ("anhydrite") of calcium sulfate.

7. The transdermal therapeutic system according to one or several of claims 1 to 6 characterized in that the estradiol is present as dispersion of an anhydrous crystallizate.

8. The transdermal therapeutic system according to one or several of claims 1 to 7 characterized in that more estradiol is comprised in the matrix base material in molecularly disperse dissolved form than corresponds to the saturation solubility.

9. The transdermal therapeutic system according to one or several of claims 1 to 8 characterized in that the proportion of the mineral water-binding additive in the total matrix material amounts to between 1 and 40%-wt., and preferably to 20%-wt.

10. The transdermal therapeutic system according to one or several of claims 1 to 9 characterized in that the matrix base material is a polymer which, under water-free conditions, has a solubility for the active substance estradiol of between 0.4 and 3.0% (w/w).

11. The transdermal therapeutic system according to claim 10 characterized in that the matrix base material is a polyacrylate polymer.

12. A process for the production of the transdermal therapeutic system according to one or several of the preceding claims, characterized by the steps:
- preparing a suspension of estradiol-semihydrate and water-binding mineral additive in a solution, dispersion, or melt of the matrix base material,
- using this suspension, applying a layer on a sheet-like substrate,
- carrying out a conversion of estradiol-semihydrate into anhydrous estradiol in the layer by heating to 90 to 175°C.

13. The process according to claim 12 characterized in that the dry layer is heated to 90 to 200°C until a conversion of the hydrous form of the mineral water-binding additive into its anhydrous form has taken place.

14. The process according' to any one of claims 12 or 13 characterized in that the transdermal therapeutic system is packed into a moistureproof package which comprises a desiccant, if required.

## Revendications

1. Système thérapeutique transdermique contenant le principe actif oestradiol et éventuellement d'autres principes actifs et éventuellement des additifs liant l'eau, avec une structure multicouche constituée d'une couche dorsale imperméable au principe actif et à l'humidité, d'une couche matricielle contenant le principe actif et le cas échéant d'une couche de protection détachable, caractérisé en ce que la matrice contient le principe actif sous forme d'oestradiol anhydre, ensemble avec un additif liant l'eau, grâce auquel on garantit une humidité d'équilibre empêchant la précipitation du semi-hydrate d'oestradiol.

2. Système thérapeutique transdermique selon la revendication 1, caractérisé en ce que la matrice contient plusieurs couches contenant du principe actif et en ce qu'au moins une de ces couches contient un additif minéral pharmaceutiquement acceptable liant l'eau.

3. Système thérapeutique transdermique selon la revendication 1 ou 2, caractérisé en ce que le matériau de la matrice contient l'additif liant l'eau sous forme d'une suspension finement dispersée.

4. Système thérapeutique transdermique selon une ou plusieurs des revendications 1 à 3, caractérisé en ce que l'additif liant l'eau est un minéral.

5. Système thérapeutique transdermique selon une ou plusieurs des revendications 1 à 4, caractérisé en ce que l'additif liant l'eau est l'anhydride d'un sel de métal alcalino-terreux ou alcalin.

6. Système thérapeutique transdermique selon une ou plusieurs des revendications 1 à 4, caractérisé en ce que l'additif liant l'eau est le semihydrate ou l'anhydride du sulfate de calcium ("anhydrite").

7. Système thérapeutique transdermique selon une ou plusieurs des revendications 1 à 6, caractérisé en ce que l'oestradiol se trouve sous forme d'une dispersion d'un produit de cristallisation anhydre.

8. Système thérapeutique transdermique selon une ou plusieurs des revendications 1 à 7, caractérisé en ce que dans le matériau de base de la matrice est contenu une quantité d'oestradiol sous forme dispersée à l'échelle moléculaire supérieure à la quantité qui correspond à la solubilité de saturation.

9. Système thérapeutique transdermique selon une ou plusieurs des revendications 1 à 8, caractérisé en ce que la proportion de l'additif minéral liant l'eau au matériau de matrice total vaut entre 1 à 40 % en poids et de préférence 20 % en poids.

10. Système thérapeutique transdermique selon une ou plusieurs des revendications 1 à 9, caractérisé en ce que le matériau de base de la matrice est un polymère qui dans des conditions anhydres présente une solubilité pour le principe actif oestradiol entre 0,4 et 3,0 % (w/w).

11. Système thérapeutique transdermique selon la revendication 10, caractérisé en ce que le matériau de base de la matrice est un polymère de polyacrylate.

12. Procédé pour la production du système thérapeutique transdermique selon une ou plusieurs des revendications précédentes, caractérisé par les étapes :
- dans une solution, dispersion ou masse en fusion du matériau de base de la matrice, on prépare une suspension du semi-hydrate d'oestradiol et de l'additif minéral liant l'eau
- on applique, au moyen de la suspension, une couche sur un matériau de support sous forme d'un film
- dans la couche, on effectue par chauffage à 90 à 175 °C une conversion du semi-hydrate d'oestradiol en oestradiol anhydre.

13. Procédé selon la revendication 12, caractérisé en ce que la couche séchée est chauffée à 90 à 200 °C jusqu'à ce qu'une conversion de la forme hydratée de l'additif minéral liant l'eau en sa forme anhydre soit réalisée.

14. Procédé selon l'une des revendications 12 ou 13, caractérisé en ce que le système thérapeutique transdermique est conditionné dans un emballage imperméable à la vapeur d'eau, celui-ci contenant le cas échéant un agent dessicatif supplémentaire.
